# EUROPEAN PATENT APPLICATION

(11) **EP 2 047 815 A1**
(43) Date of publication of application: **15.04.2009**
(21) Application number: 07019836.1
(22) Date of filing: 10.10.2007
(51) Int. Cl.: A61B 17/84, A61B 17/86

(54) **A spinal fixation device having a flexible cable and jointed components received thereon**

(71) Applicant: Yeh, Chung-Chun, Taipei (TW)
(72) Inventor: Yeh, Chung-Chun, Taipei (TW)
(74) Representative: Beetz & Partner

(57) **Abstract**

The invention relates to a spinal fixation device which includes a flexible cable 20; a plurality of components 30 disposed on the flexible cable 20; and a plurality of fixing nails 40, wherein the flexible cable 20 may be movingly received by the fixing nails 40 after it has been fitted with the components 30 , and a screw 50 may be used to secure the flexible cable 20 along with the components 30 onto the fixing nails 40; wherein two neighboring components 30 disposed on the flexible cable 20 are connected with an adjustable angle.

## Description

### FIELD OF THE INVENTION

The invention relates to a spinal fixation device, and more particularly to a spinal flexible-cable fixation device comprising a flexible cable and a plurality of components jointly fitted on the flexible cable, so that an inflexible section covered by the components is formed while retaining a flexible section thereof which is not covered by the components, wherein two neighboring components are connected with an adjustable angle.

### BACKGROUND OF THE INVENTION

Traditionally, if a spinal injury is mended by using nails and rods, a fixed rod made of titanium alloys or stainless steel is used in combination with a plurality of nails that are implanted into several vertebrae, so that the injured vertebrae are forcefully connected and secured to one another.

However, such method of forcefully securing the vertebrae together is not only unable to reduce undue stress on the vertebrae, but also unable to stimulate the injured vertebrae so as to promote the growth of bone mass and stabilize the injured vertebrae. As a result, an afflicted patient is either bedridden or only able to get around with a support for a long time, and the injured spine may not recover soon enough.

On the other hand, if the implanted nails are connected by using rods made of flexible cables only, it gives rise to two kinds of problems consequently. One of which is that the flexible cables may be too hard and stiff, which causes this remedy to have the same disadvantages as that from using the fixed rod made of titanium alloys or stainless steel. The other problem is that if the flexible cable is too flimsy, it cannot provide adequate support for the spine and the flexible cable is prone to deformation. Moreover, if the flexible cable is enclosed with a supporting tube, the flexible cable would become substantially weakened in regard to its flexibility, elasticity, and bending property, which makes this approach have the same disadvantages as that of using an ordinary rod.

### SUMMARY OF THE INVENTION

In light of the aforesaid problems, a spinal fixation device for securing the nails implanted into vertebrae and providing support to the spine is proposed in the invention, which has a flexible cable with adequate flexibility and elasticity, and a plurality of components fitted onto the flexible cable, wherein the flexible cable has flexible sections and inflexible sections that are separated from each other by the components jointly fitted on the flexible cable. The components have contacts that form an adjustable angle between two neighboring components so as to ensure the flexibility of the flexible cable. Therefore, a primary objective of the invention is to propose a spinal fixation device that comprises a flexible cable and a plurality of components jointly fitted onto the flexible cable.

This objective is solved by a spinal fixation device comprising:
a flexible cable;
a plurality of components fittingly disposed on the flexible cable; and/or a plurality of fixing nails having a fixing portion at an end thereof,
   wherein each fixing portion has a screw hole and an opening, the flexible cable can be movably placed into the opening after being fitted with the components, and a screw can be screwed into the screw hole subsequently so as to secure the flexible cable along with the components in each fixing nail.

Sections of the flexible cable enclosed by the components are inflexible, whereas sections of the flexible cable not enclosed by the components remain flexible, and/or two neighboring components disposed on the flexible cable are connected with an adjustable angle, while the fixing nails are linked together by the flexible cable fitted with the components.

Each of the two neighboring components has two spherical ends, and the two neighboring components are connected with the spherical ends thereof contacting each other, so that the two neighboring components disposed on the flexible cable are connected with an adjustable angle.

Each of the components is a cylinder with spherical ends at both ends thereof.

Each of the components is in the form of a sphere.

A contact between the two neighboring components is formed by a spherical end and a matching indentation thereof respectively, so that the two neighboring components disposed on the flexible cable are connected with the contact formed by the spherical end and the matching indentation.

Each of the components is a cylinder with the spherical end at one end and the matching indentation at another end thereof.

Each of the components is a concave body with the spherical end at one end and the matching indentation at another end thereof.

A contact between the two neighboring components is formed by two arcuate surfaces thereof, so that the two neighboring components disposed on the flexible cable are connected with with the contact formed by the two arcuate surfaces.

Each of the components is a cylinder with the arcuate surfaces provided at both ends thereof.

A contact between the two neighboring components is formed by a conical end and a matching indentation thereof respectively, so that the two neighboring components disposed on the flexible cable are connected with the contact formed by the conical end and the matching indentation.

Each of the components is a cylinder having the conical end and the matching indentation at another end thereof.

Each of the components is a concave body with the conical end at one end and the matching indentation at another end thereof.

Each of the components has a hole and a slit extending from the hole, and the flexible cable is received in the hole with the slit being elastically expanded, thus allowing the components to grasp the flexible cable.

The flexible cable has a retainer further disposed at one end or both ends thereof; when one end of the flexible cable is pulled, the retainer at another end of the flexible cable presses against the fixing portion of the fixing nail, so as to prevent the flexible cable from moving further.

The flexible cable and the retainer are integrally formed.

The screw has a protrusion disposed on a bottom surface thereof, and the protrusion is adapted to press against and secure the flexible cable onto the fixing nail.

The flexible cable is a wire cable with multiple strands of wires which are twisted, and the components are provided with holes, through which the flexible cable is enclosed by the components.

The fixing portion has the screw hole disposed vertically and a U-shaped groove disposed horizontally therein, the flexible cable can be movably placed into the U-shaped groove after being fitted with the components, and the screw is screwed into the screw hole subsequently so as to secure the flexible cable along with the components in each fixing nail.

The fixing nail further comprises a nail portion detachable to the fixing portion, the nail portion has the screw hole disposed vertically and a U-shaped groove disposed horizontally therein, the nail portion has a screw end and a spherical end; the screw end is fitted through the screw hole with the spherical end being subsequently secured in the U-shaped groove of the fixing portion; the flexible cable can be movably placed into the U-shaped groove after being fitted with the components, and the screw is movably screwed into the screw hole, thus securing the fixing portion, the nail portion, and the flexible cable together.

The screw hole of the fixing nail is a slanted screw hole, and the fixing portion further includes a fitting groove that is obliquely open to the screw hole; the flexible cable is received in the fitting groove of the fixing portion, and the screw is screwed into the slanted screw hole, thus securing the fixing nail and the flexible cable together.

The fixing nail has a through hole that is open to the screw hole; the flexible cable is fitted into the fixing portion via the through hole, and the screw is used to secure the fixing nail and the flexible cable together.

A plurality of conversion components is disposed onto the flexible cable, each of the conversion components has a middle portion and two linking portions extending from both ends thereof, the middle portion matches the opening of the fixing portion, and the linking portions are adapted to be connected to the component with an adjustable angle.

A bent insertion kit that has a bent tube and a flexible needle are provided;
wherein the flexible cable that has been fitted with the components is able to be placed into the bent tube, and the flexible needle is then inserted into the bent tube, so as to guide and align the flexible cable into the opening of the fixing portion of the fixing nail.

The above features can be combined in any way partly or as a whole.

### BRIEF DESCRIPTION OF DRAWINGS

The structure and the technical means adopted by the present invention to achieve the above and other objectives can be best understood by referring to the following detailed description of the preferred embodiments and the accompanying diagrams, wherein:
Figs. 1a and 1b are schematic views that show a spinal fixation device comprising a flexible cable and components before and after being assembled according to a preferred embodiment of the present invention, respectively.
Fig. 1c is a schematic view that shows a flexible cable integrally formed with a retainer at one end thereof suitable for use in the spinal fixation device shown in Figs. 1a and 1b.
Fig. 2 is a schematic view that shows the conversion component 31 in Figs. 1a and 1b.
Figs. 3a and 3b are schematic views that show components 30 with two spherical ends 302 of the spinal fixation device of the present invention, wherein the components 30 shown in Fig. 3 a are generally sphere in shape and the components 30 shown in Fig. 3b are generally cylindrical in shape
Figs. 3c to 3e are schematic views that show components 32 of the spinal fixation device of the present invention with one spherical end, a matching indentation at another end thereof, and a various length between the two ends.
Fig. 3f are schematic views that show components of the spinal fixation device of the present invention, which are generally flattened with arcuate surfaces at both ends.
Fig. 3g are schematic views that show components of the spinal fixation device of the present invention, which are conical at one end and has a matching indentation at another end, with a various length between the two ends.
Figs. 4 to 6 are schematic views that show the spinal fixation device of the present invention using fixing nails of different designs.
Fig. 7 is a schematic view that shows the implantation of the spinal fixation device into vertebrae according to the present invention.
Fig. 8a is a schematic view that shows a bent insertion kit for inserting the flexible cable together with the components into the fixing portion of the fixing nails of the present invention.
Fig. 8b is a schematic view that shows the usage of the bent insertion kit in Fig. 8a for implanting the spinal fixation device of the present invention.

### DETAILED DESCRIPTION OF PREFERRED EMBODIMENTS

Preferred embodiments and drawings of the invention are given below to facilitate better understanding toward the invention.

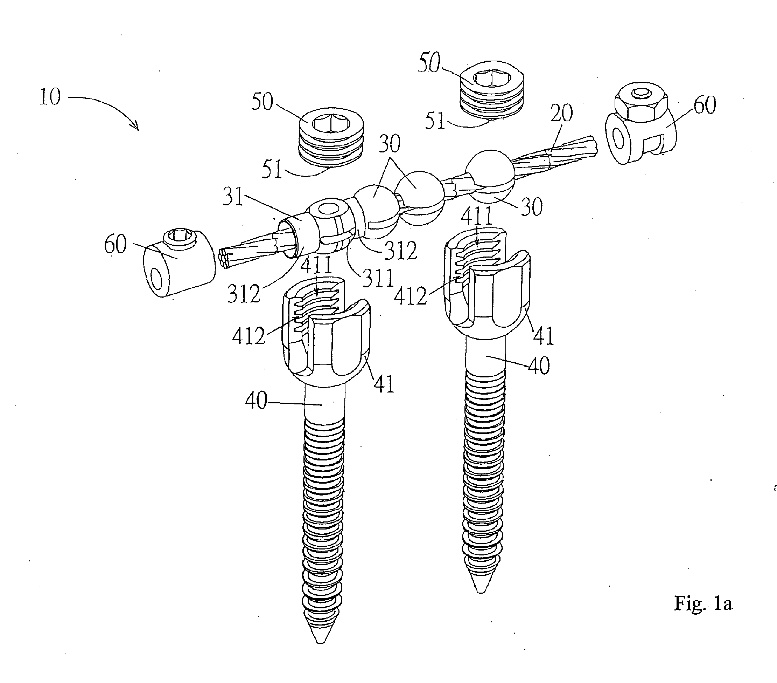

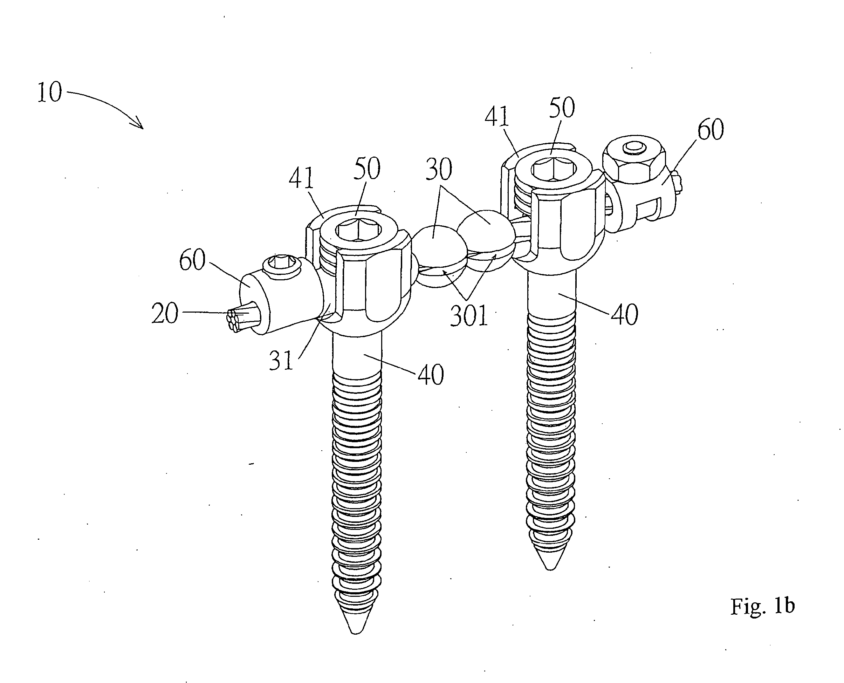

Figs. 1a and 1b show a spinal fixation device 10 comprising a flexible cable and components according to a first preferred embodiment of the invention, which comprises a flexible cable 20, a plurality of components 30 formed into a first shape, a plurality of conversion components 31 (only one is shown in the drawings), a plurality of fixing nails 40, a plurality of screws 50, and a plurality of retainers 60.

The flexible cable 20 is a wire cable that has elasticity and flexibility resulted from material and structure thereof, and when the flexible cable 20 is bent, compressed, or stretched; the flexible cable 20 may be contorted in response to the applied forces. Each of the components 30 has a slit 301 disposed thereof, and the flexible cable 20 may be fittingly disposed through a central opening as well as a narrow gap of the slit 301, so that the components 30 are elastically fitted onto the flexible cable 20.

Each of the conversion components 31 has a middle portion 311, respectively; the middle portion 311 is functionally similar to the components 30, and may be fitted onto the flexible cable 20 and disposed into the fixing nails 40; the middle portion 311 has a linking portion 312 extendingly disposed at both ends thereof, and the linking portions 312 may movably contact with the components 30. Each of the fixing nails 40 has a fixing portion 41, respectively; the fixing portion 41 has a screw hole 411 vertically disposed therein and a U-shaped groove 412 horizontally disposed therein. When the flexible cable 20 is fitted with the components 30 or the conversion components 31, the flexible cable 20 may be movably placed into the U-shaped groove 412. The screws 50 are screwed into the screw holes 411, so as to tightly secure the fixing nails 40 together with the combination of the flexible cable 20 and the components 30 or the conversion components 31.

Sections of the flexible cable 20 are enclosed by the combination of the conversion components 31 that extendingly linked to the components 30, as well as the combination of the components 30 that movably contact one another. Because the components 30 are hard and may support each other, the aforesaid sections of the flexible cable 20 are limited by the components 30 and are relatively difficult to be bent and compressed.

On the other hand, other sections of the flexible cable 20 that are not enclosed by the components 30 and are free from any supports or restrictions (such as the components 30), thus these sections of the flexible cable 20 may be flexibly bent, compressed, or stretched. Moreover, two neighboring components 30 are connected with an adjustable angle; an adjustable angle may be formed when a surface of a sphere comes into contact with a surface of another sphere. In addition, the fixing nails 40 are connected to each other with the flexible cable 20 that has been fitted with the components 30.

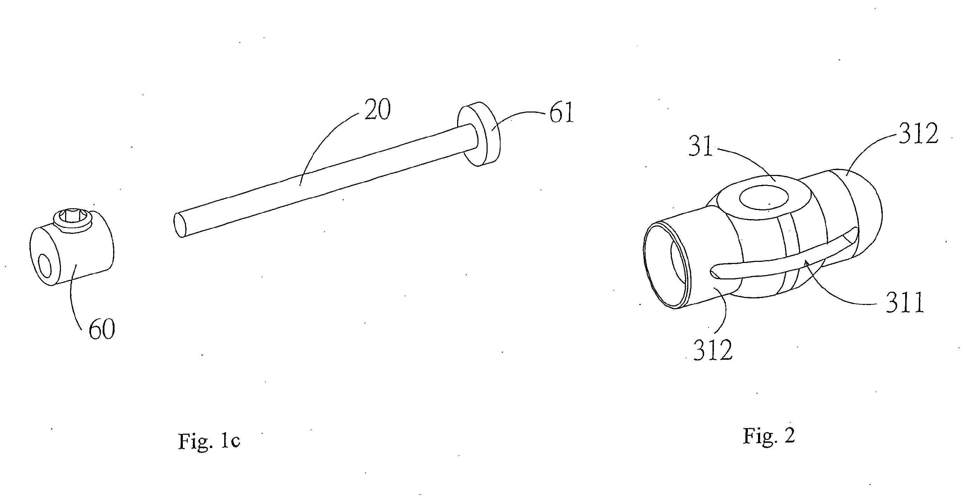

The screw 50 has a protrusion 51 disposed on a bottom surface thereof, and the protrusion 51 may press against and secure the combination of the flexible cable 20 and the components 30 or the conversion components 31 onto the fixing nails 40. Furthermore, the retainers 60 are disposed at both ends of the flexible cable 20, so that when one end of the flexible cable 20 is pulled, the retainer 60 at another end of the flexible cable 20 may press against the fixing portion 41 of the fixing nail 40, and subsequently prevent the flexible cable 20 from moving further. In contrast, if the flexible cable 20 is made of special materials like the polyetheretherketone (PEEK) plastic, a retainer 61 shaped into a different form may be directly formed at an end of the flexible cable 20, which achieves identical purpose as the aforesaid retainer 60 disposed at the other end of the flexible cable 20, as shown in Fig. 1c.

Details of the conversion component 31 is shown in Fig. 2, wherein the middle portion 311, which performs a similar function as that of the aforesaid component 30 and may be fitted onto the flexible cable 20. The conversion component 31 is shaped such that allows it to be more fittingly disposed into the fixing nail 40. The linking portion 312 extending from both ends of the conversion component 31 may be either spherical end or recessed end, and contacts the components 30 movably with an adjustable angle.

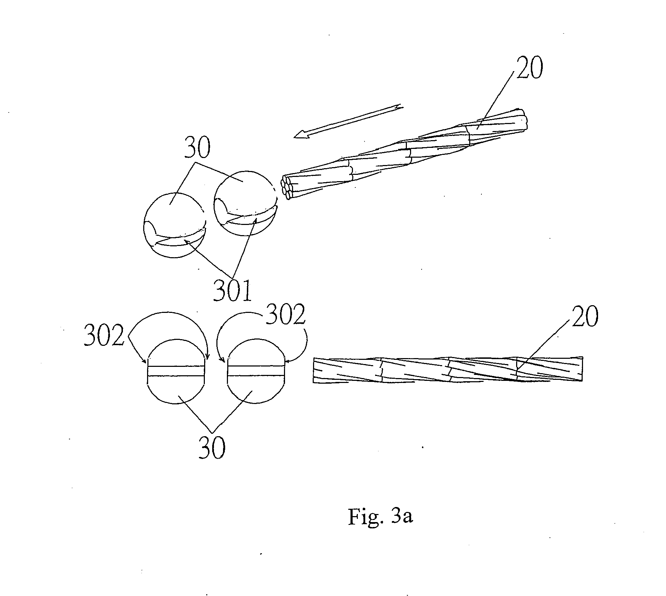

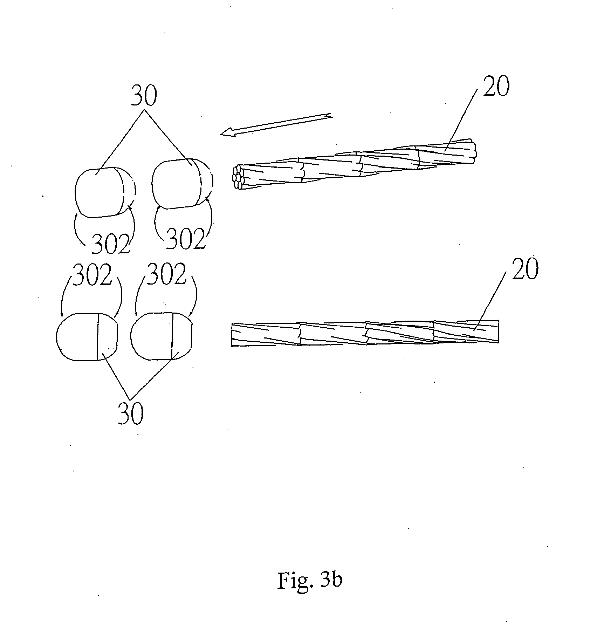

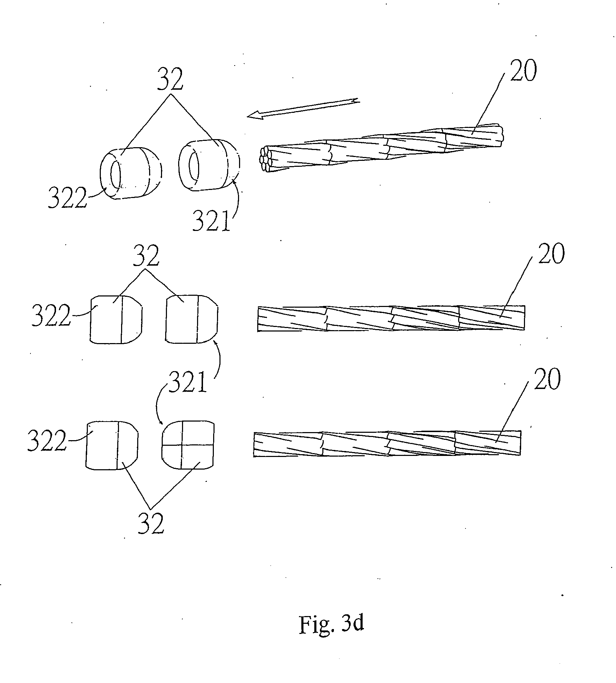

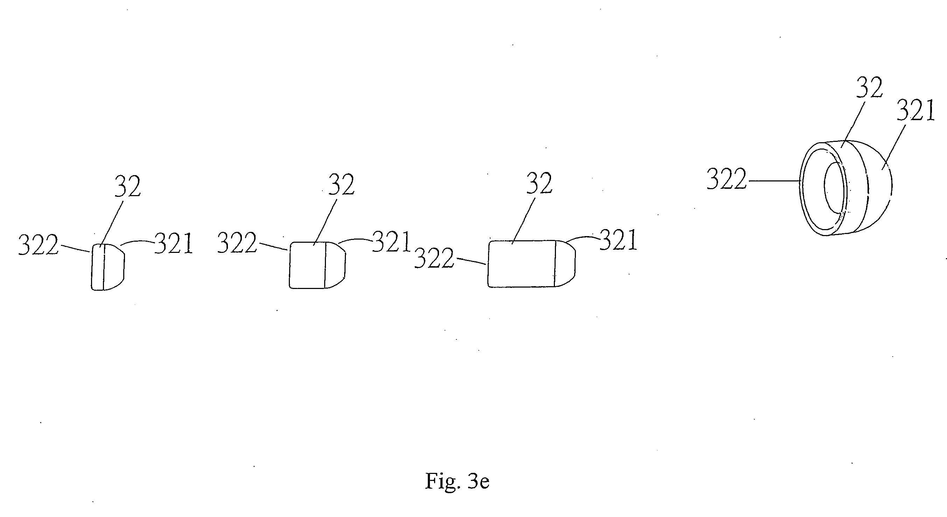

Figs. 3a and 3b show the components 30 with two spherical ends 302 according to the first preferred embodiment of the invention, wherein the components 30 shown in Fig. 3a are generally sphere in shape and the components 30 shown in Fig. 3b are generally cylindrical in shape

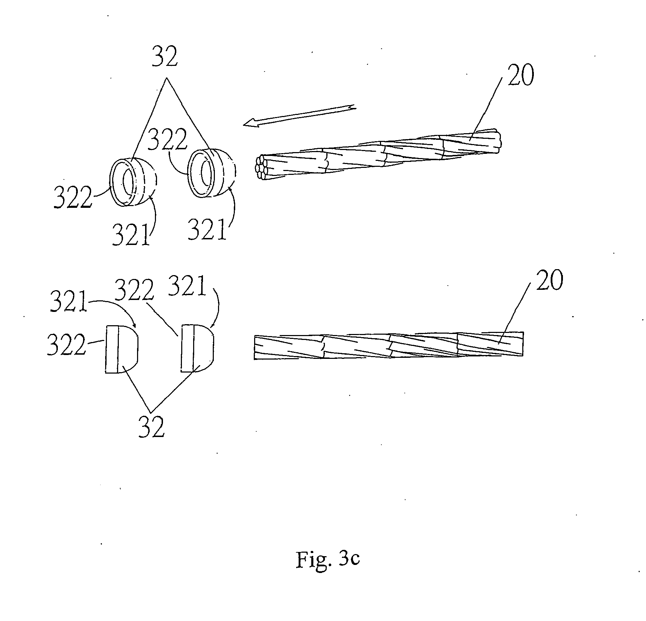

Figs. 3c to 3e show the components 32 with one spherical end 321 and one matching indention 322 at the other end according to a second preferred embodiment of the invention, and how the components 32 contact one another with an adjustable angle. If a spherical end 321 contacts a matching indentation 322, each of the components 32 may be an elongated cylinder as a whole without being limited in length. Moreover, if the elongated cylindrical component 32 is thin and short, it may be shaped as a concave body in which a lateral end thereof being the spherical end 321, and an opposite lateral end thereof being the matching indentation 322, so that one component 32 may be arranged adjacent to another component 32 in a scale-like fashion. A plurality of components 32 may be arranged such that a spherical end 321 faces another spherical end 321.

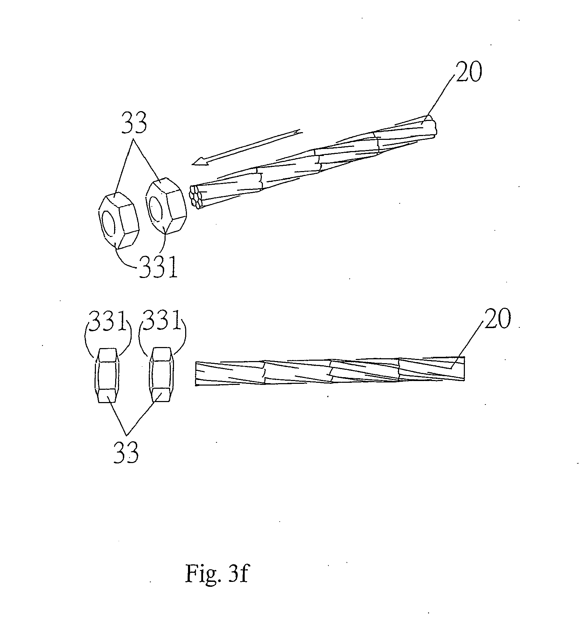

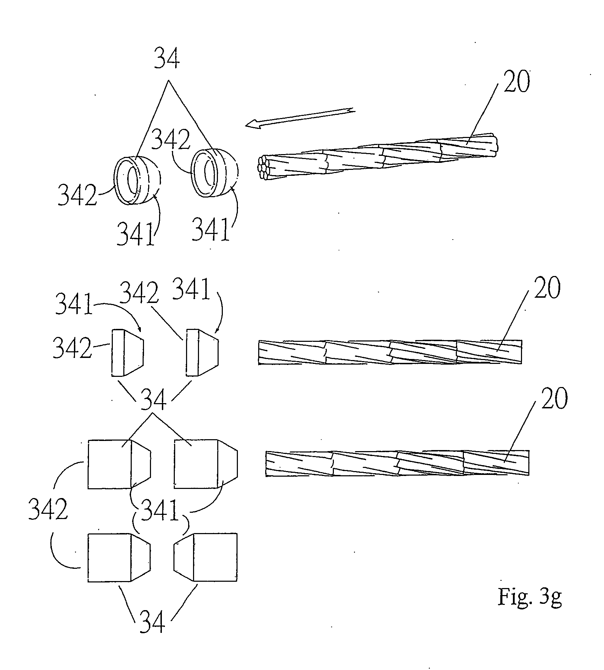

Figs. 3f and 3g show the components 33 and 34 according to a third and a fourth preferred embodiments of the invention, and how the components 33 and 34 contact one another with an adjustable angle. In Fig. 3f, the components 33 are generally flattened with arcuate surfaces 331 at both ends, so that the components 33 can be movably connected to each other with an adjustable angle. In Fig. 3g, a conical end 341 contacts a matching indentation 342 between two adjacent components 34, or the conical ends 341 of two adjacent components contact each other, so that the components 33 can be movably connected to each other with an adjustable angle. The components 34 may have different lengths between the two ends.

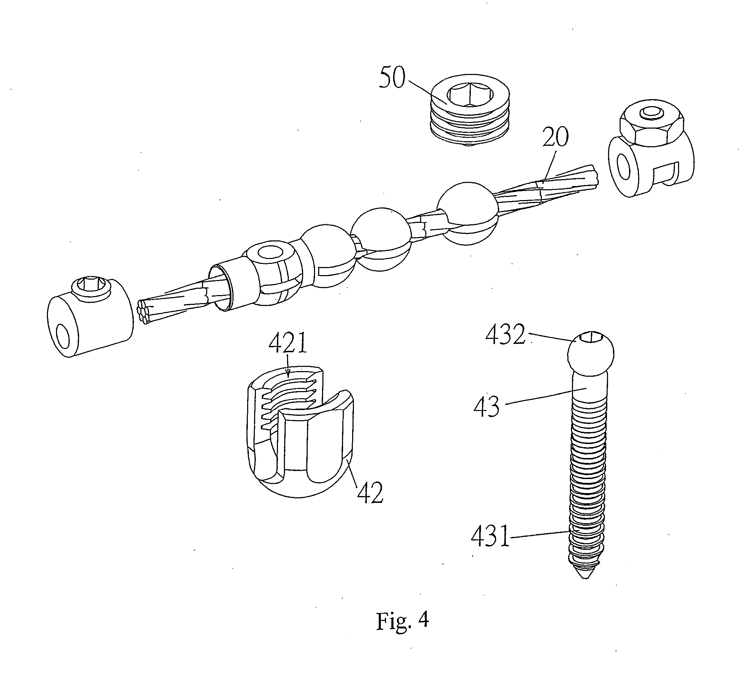

Fig. 4 shows that each of the fixing nails 40 may be further divided into a fixing portion 42 and a nail 43 that movably match each other. The fixing portion 42 has a screw hole 421 disposed therethrough, while the nail 43 has a screw end 431 and a spherical end 432; the screw end 431 may be fitted through the screw hole 421, and the spherical end 432 is retained in the fixing portion 42. The screws 50 may be movably screwed into the screw hole 421 so as to press and secure combination between the fixing portion 42, the nail 43, and the flexible cable 20.

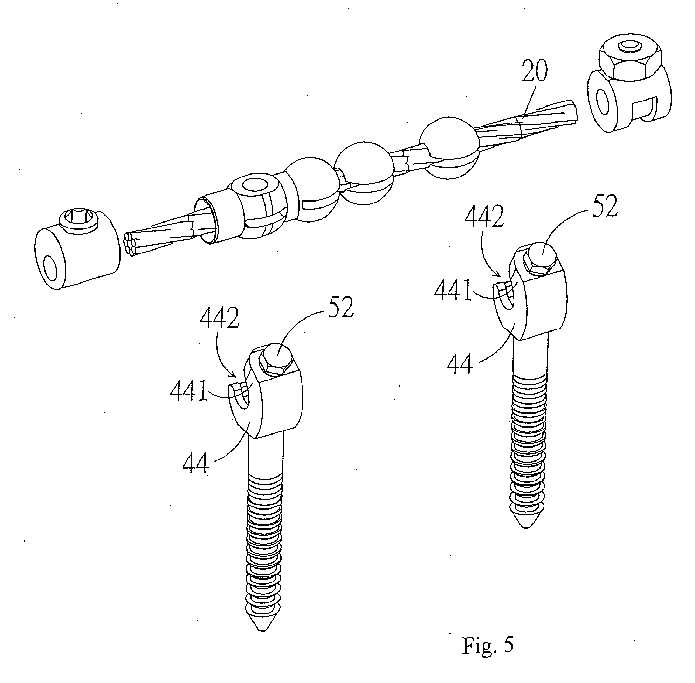

Fig. 5 shows another preferred embodiment of the fixing nail 40, in which a screw hole of a fixing portion 44 thereof may be further altered into a slanted screw hole 441, and the fixing nail 40 has a fitting groove 442 that is obliquely open to the slanted screw hole 441. Once the flexible cable 20 is placed into the fixing portion 44 through the fitting groove 442, a complementary screw 52 may be screwed into the slanted screw hole 441 and consequently secures combination between the fixing nail 40 and the flexible cable 20.

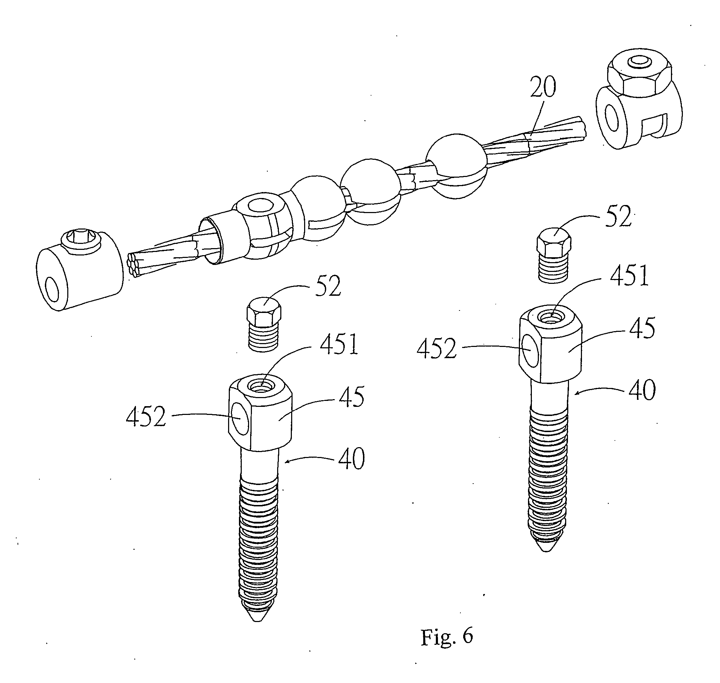

Fig. 6 shows another fixing portion 45 for the fixing nail 40 according to another preferred embodiment of the invention, in which a screw hole 451 is openly connected to an opening 452, and the flexible cable 20 may be fitted into the fixing portion 45 via the opening 452, while the screw 52 may be screwed into the screw hole 451 and hence secures combination between the fixing nail 40, and the flexible cable 20.

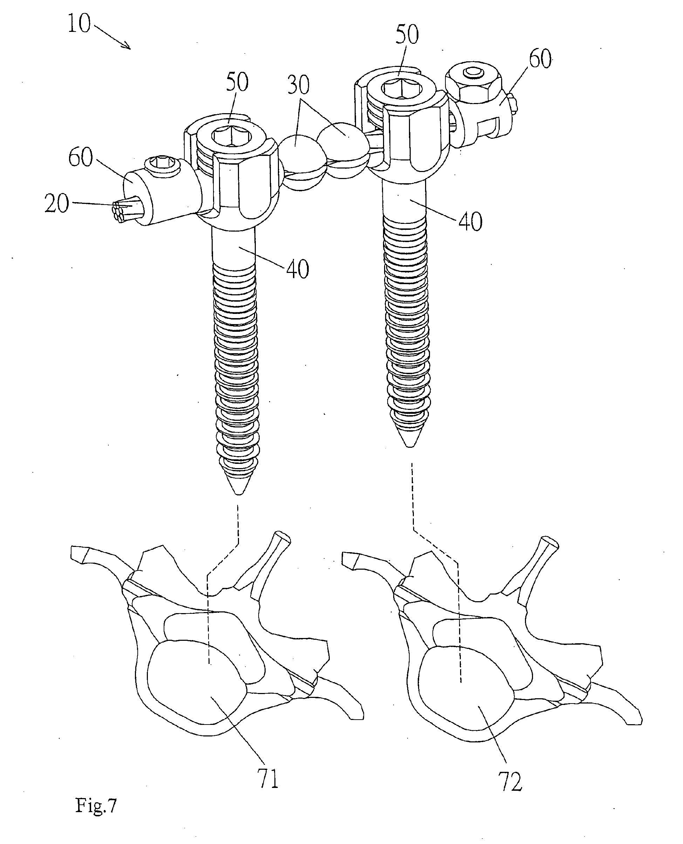

Fig. 7 shows that a spinal fixation device 10 of the present invention is implanted into vertebrae 71 and 72. The sections of the flexible cable 20 that are enclosed by the components 30 are inflexible, whereas the sections of the flexible cable 20 that are not enclosed by the components 30 are flexible. The flexible cable 20 may be slightly bent with an adjustable angle between two neighboring components 30, and the fixing nails 40 are linked together by the flexible cable 20.

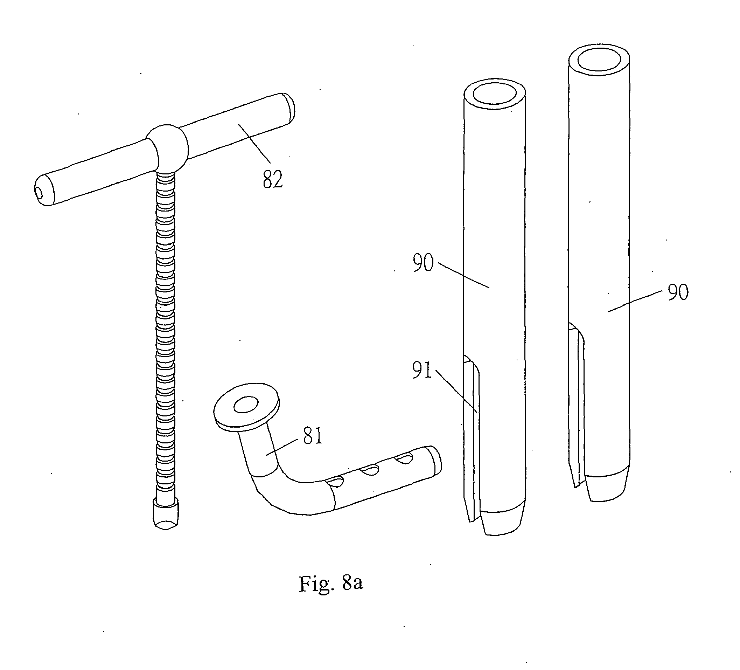

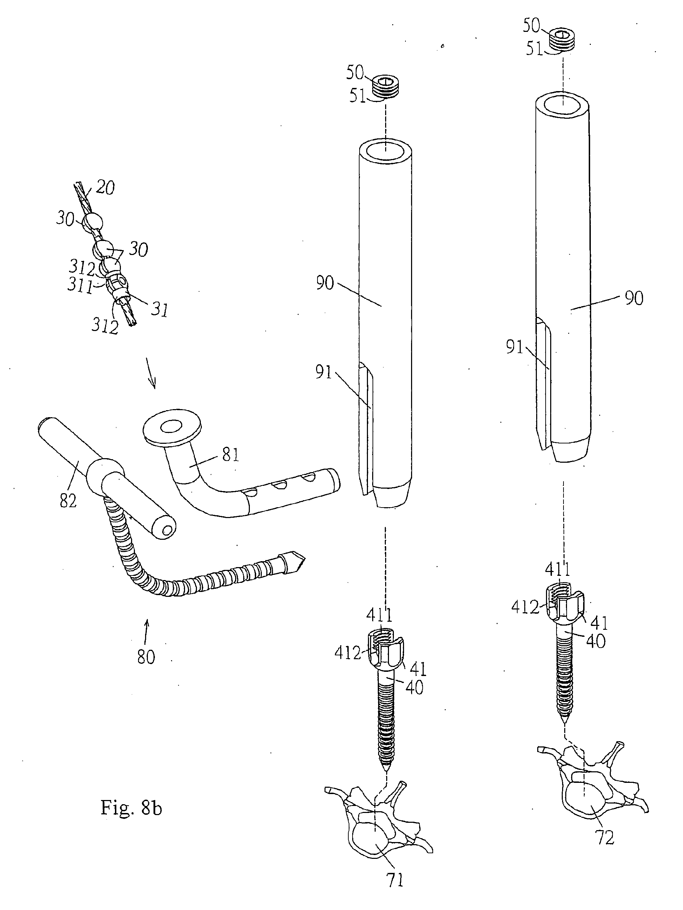

As shown in Figs. 8a and 8b, the spinal fixation device 10 is installed with the help from a bent insertion kit 80 and a tube kit 90. The bent insertion kit 80 has a bent tube 81 and a flexible needle 82; and the flexible needle 82 can be flexibly bent along curvature of the bent tube 81 and placed therein. The bent insertion kit 80 may be inserted from outside of a human body. The tubes of the tube kit 90 allows the nails 40 and screws 50 to be fit into the vertebrae 71 and 72 through the longitudinal holes thereof, and they also have a lateral groove 91. The insertion kit 80 guide and align the flexible cable 20, that has been fitted with the components 30 and the conversion components 31, through the lateral grooves 91 into the

U-shaped grooves 412 of the fixing portions 41 of the fixing nails 40.

Although a preferred embodiment of the invention has been described for purposes of illustration, it is understood that various changes and modifications to the described embodiment can be carried out without departing from the scope and the spirit of the invention as disclosed in the appended claims.

## Claims

1. A spinal fixation device, comprising:
a flexible cable (20);
a plurality of components (30) fittingly disposed on the flexible cable (20);
and
a plurality of fixing nails (40) having a fixing portion (41) at an end thereof,
wherein each fixing portion (41) has a screw hole (411) and an opening, the flexible cable (20) can be movably placed into the opening after being fitted with the components (30), and a screw (50) can be screwed into the screw hole (411) subsequently so as to secure the flexible cable (20) along with the components (30) in each fixing nail (40);
**characterized in that**: sections of the flexible cable (20) enclosed by the components (30) are inflexible, whereas sections of the flexible cable (20) not enclosed by the components (30) remain flexible, and two neighboring components (30) disposed on the flexible cable (20) are connected with an adjustable angle, while the fixing nails (40) are linked together by the flexible cable (20) fitted with the components (30).

2. The spinal fixation device according to claim 1, wherein each of the two neighboring components has two spherical ends, and the two neighboring components are connected with the spherical ends thereof contacting each other, so that the two neighboring components disposed on the flexible cable are connected with an adjustable angle.

3. The spinal fixation device according to claim 1 or 2, wherein each of the components is a cylinder with spherical ends at both ends thereof.

4. The spinal fixation device according to at least one of claims 1 to 3,
wherein each of the components is in the form of a sphere.

5. The spinal fixation device according to at least one of claims 1 to 4, wherein a contact between the two neighboring components is formed by a spherical end and a matching indentation thereof respectively, so that the two neighboring components disposed on the flexible cable are connected with the contact formed by the spherical end and the matching indentation.

6. The spinal fixation device according to at least one of claims 1 to 5, wherein each of the components is a cylinder with the spherical end at one end and the matching indentation at another end thereof.

7. The spinal fixation device according to at least one of claims 1 to 6, wherein each of the components is a concave body with the spherical end at one end and the matching indentation at another end thereof.

8. The spinal fixation device according to at least one of claims 1 to 7, wherein a contact between the two neighboring components is formed by two arcuate surfaces thereof, so that the two neighboring components disposed on the flexible cable are connected with with the contact formed by the two arcuate surfaces.

9. The spinal fixation device according to at least one of claims 1 to 8, wherein each of the components is a cylinder with the arcuate surfaces provided at both ends thereof.

10. The spinal fixation device according to at least one of claims 1 to 9,
wherein a contact between the two neighboring components is formed by a conical end and a matching indentation thereof respectively, so that the two neighboring components disposed on the flexible cable are connected with the contact formed by the conical end and the matching indentation.

11. The spinal fixation device according to at least one of claims 1 to 10, wherein each of the components is a cylinder having the conical end and the matching indentation at another end thereof.

12. The spinal fixation device according to at least one of claims 1 to 11, wherein each of the components is a concave body with the conical end at one end and the matching indentation at another end thereof.

13. The spinal fixation device according to at least one of claims 1 to 12, wherein each of the components has a hole and a slit extending from the hole, and the flexible cable is received in the hole with the slit being elastically expanded, thus allowing the components to grasp the flexible cable.

14. The spinal fixation device according to at least one of claims 1 to 13, wherein the flexible cable has a retainer further disposed at one end or both ends thereof; when one end of the flexible cable is pulled, the retainer at another end of the flexible cable presses against the fixing portion of the fixing nail, so as to prevent the flexible cable from moving further.

15. The spinal fixation device according to at least one of claims 1 to 14,
wherein the flexible cable and the retainer are integrally formed.

16. The spinal fixation device according to at least one of claims 1 to 15, wherein the screw has a protrusion disposed on a bottom surface thereof, and the protrusion is adapted to press against and secure the flexible cable onto the fixing nail.

17. The spinal fixation device according to at least one of claims 1 to 16, wherein the flexible cable is a wire cable with multiple strands of wires which are twisted, and the components are provided with holes, through which the flexible cable is enclosed by the components.

18. The spinal fixation device according to at least one of claims 1 to 17, wherein the fixing portion has the screw hole disposed vertically and a U-shaped groove disposed horizontally therein, the flexible cable can be movably placed into the U-shaped groove after being fitted with the components, and the screw is screwed into the screw hole subsequently so as to secure the flexible cable along with the components in each fixing nail.

19. The spinal fixation device according to at least one of claims 1 to 18, wherein the fixing nail further comprises a nail portion detachable to the fixing portion, the nail portion has the screw hole disposed vertically and a U-shaped groove disposed horizontally therein, the nail portion has a screw end and a spherical end; the screw end is fitted through the screw hole with the spherical end being subsequently secured in the U-shaped groove of the fixing portion; the flexible cable can be movably placed into the U-shaped groove after being fitted with the components, and the screw is movably screwed into the screw hole, thus securing the fixing portion, the nail portion, and the flexible cable together.

20. The spinal fixation device according to at least one of claims 1 to 19, wherein the screw hole of the fixing nail is a slanted screw hole, and the fixing portion further includes a fitting groove that is obliquely open to the screw hole; the flexible cable is received in the fitting groove of the fixing portion, and the screw is screwed into the slanted screw hole, thus securing the fixing nail and the flexible cable together.

21. The spinal fixation device according to at least one of claims 1 to 20, wherein the fixing nail has a through hole that is open to the screw hole; the flexible cable is fitted into the fixing portion via the through hole, and the screw is used to secure the fixing nail and the flexible cable together.

22. The spinal fixation device according to at least one of claims 1 to 21, further comprising a plurality of conversion components disposed onto the flexible cable, each of the conversion components has a middle portion and two linking portions extending from both ends thereof, the middle portion matches the opening of the fixing portion, and the linking portions are adapted to be connected to the component with an adjustable angle.

23. The spinal fixation device according to at least one of claims 1 to 22, further comprising a bent insertion kit that has a bent tube and a flexible needle; wherein the flexible cable that has been fitted with the components is able to be placed into the bent tube, and the flexible needle is then inserted into the bent tube, so as to guide and align the flexible cable into the opening of the fixing portion of the fixing nail.
